(19) Europäisches Patentamt
European Patent Office
Office européen des brevets

(11) Numéro de publication : **0 018 893**
**B1**

(12) # FASCICULE DE BREVET EUROPÉEN

(45) Date de publication du fascicule du brevet : **26.01.83**

(51) Int. Cl.³ : **C 07 C 35/06**, C 07 C 35/48, C 07 C103/19, C 07 C121/48

(21) Numéro de dépôt : **80400556.9**

(22) Date de dépôt : **24.04.80**

(54) **Nouveaux dérivés de l'alléthrolone et leur procédé de préparation.**

(30) Priorité : **26.04.79 FR 7910650**

(43) Date de publication de la demande : **12.11.80 Bulletin 80/23**

(45) Mention de la délivrance du brevet : **26.01.83 Bulletin 83/04**

(84) Etats contractants désignés : **AT BE CH DE FR GB IT LI LU NL SE**

(56) Documents cités :
**FR A 1 505 423**
**FR A 2 213 013**
**FR A 2 360 546**
**FR E 93 112**

**CHEMICAL ABSTRACTS, vol. 86, no. 13, 28 mars 1977, page 490, no. 89226z Columbus, Ohio, U.S.A. T. SHONO et al. : « Electroorganic chemistry. XXIV. Reductive cyclization of nonconjugated acetylenic ketones to 2-methylenecyclopentanols ».**

(73) Titulaire : **ROUSSEL-UCLAF**
**35, boulevard des Invalides**
**F-75007 Paris (FR)**

(72) Inventeur : **Martel, Jacques**
**15, rue Douvillez**
**F-93140-Bondy (FR)**
Inventeur : **Tessier, Jean**
**30, rue Jean Moulin**
**F-94300-Vincennes (FR)**
Inventeur : **Teche, André**
**51, avenue Joliot Curie**
**F-92000-Nanterre (FR)**

(74) Mandataire : **Douetteau, Pierre et al**
**Boîte postale no 9 102, route de Noisy**
**F-93230-Romainville (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Nouveaux dérivés de l'alléthrolone et leur procédé de préparation

La présente invention concerne de nouveaux dérivés de l'alléthrolone et leur procédé de préparation.

L'invention a pour objet les composés de formule générale I :

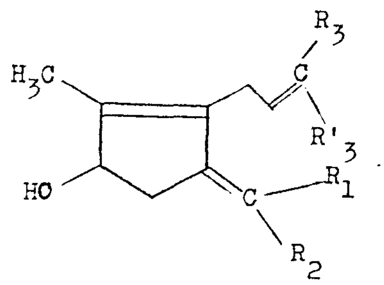

(I)

dans laquelle $R_1$ et $R_2$, identiques ou différents, représentent un groupement carbamoyle ou bien des groupements $R'_1$ et $R'_2$, lesquels, identiques ou différents, représentent un atome d'hydrogène, un atome d'halogène, un radical alcoyle comportant de 1 à 6 atomes de carbone, un radical aryle comportant de 6 à 10 atomes de carbone, un radical aralcoyle comportant de 7 à 13 atomes de carbone, un groupement alcoyloxycarbonyle comportant de 2 à 5 atomes de carbone ou un groupement cyano, $R_3$ et $R'_3$, identiques ou différents, représentent un atome d'hydrogène ou un radical alcoyle saturé ou non, comportant de 1 à 3 atomes de carbone, les composés de formule (I) pouvant être de configuration S, R, ou racémique.

Dans les composés de l'invention, $R'_1$ et $R'_2$ peuvent présenter notamment un atome de fluor, de chlore, de brome, un radical méthyle, éthyle, propyle linéaire ou ramifié, un radical phényle, naphtoyle, benzyle, un groupement alcoyloxycarbonyle dont le groupement alcoyle peut être un radical méthyle, éthyle, propyle linéaire ou ramifié, butyle linéaire ou ramifié et $R_3$ et $R'_3$ peuvent représenter notamment un atome d'hydrogène, un radical méthyle, éthyle ou propyle linéaire ou ramifié, vinyle, allyle ou propen-1-yle.

L'invention a, en particulier, pour objet les composés de formule I dont les noms suivent :

— le 1S-hydroxy 2-méthyl 3-allyl 4-méthylène cyclopent-2-ène,
— le 1RS-hydroxy 2-méthyl 3-(3-méthyl 2-buténył) 4-méthylène cyclopent-2-ène,
— le 1S-hydroxy 2-méthyl 3-allyl 4-dichlorométhylène cyclopent-2-ène,
— le 1S-hydroxy 2-méthyl 3-allyl 4-dicyanométhylène cyclopent-2-ène,
— le 1S-hydroxy 2-méthyl 3-allyl 4-éthylidène cyclopent-2-ène,
— le 1S-hydroxy 2-méthyl 3-allyl 4E-benzylidène cyclopent-2-ène,
— le 1S-hydroxy 2-méthyl 3-allyl 4-fluorochlorométhylène cyclopent-2-ène,
— le 1S-hydroxy 2-méthyl 3-allyl 4-cyanométhylène cyclopent-2-ène.
— le 1S-hydroxy 2-méthyl 3-allyl 4-(cyanoéthoxycarbonyl) méthylène cyclopent-2-ène.

L'invention a également pour objet un procédé de préparation des composés de formule I, caractérisé en ce que l'on soumet, au sein d'un solvant, un composé de formule générale II :

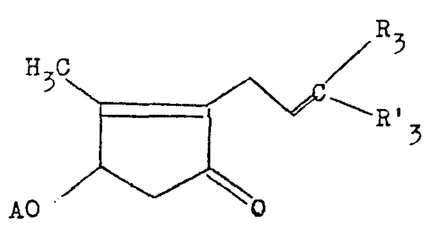

(II)

dans laquelle $R_3$ et $R'_3$ conservent les significations précitées et A représente un atome d'hydrogène, un reste d'acide carboxylique aliphatique comportant de 2 à 6 atomes de carbone, un reste d'acide borique ou un reste d'éther comportant de 2 à 6 atomes de carbone, à l'action d'un réactif de formule III :

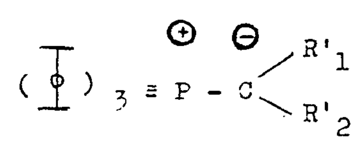

(III)

dans laquelle $R'_1$ et $R'_2$ conservent les significations précitées, débloque, le cas échéant, la fonction

hydroxyle, à l'aide d'un agent de désacylation ou de déséthérification, pour obtenir un composé de formule I désiré, dans lequel $R_1$ et $R_2$ ont les valeurs de $R'_1$ et $R'_2$, $R'_1$ et $R'_2$ étant définis comme ci-dessus, puis, le cas échéant, fait réagir un composé I dans lequel $R'_1$ et/ou $R'_2$ représentent un groupement alcoyloxycarbonyle comportant de 2 à 5 atomes de carbone et dans lequel le groupement hydroxyle est éventuellement bloqué par un reste d'acide borique ou d'acide carboxylique aliphatique comportant de 2 à 6 atomes de carbone ou par un reste d'éther comportant de 2 à 6 atomes de carbone, avec l'ammoniac, puis débloque, le cas échéant, le groupement hydroxyle pour obtenir le composé I désiré, dans lequel $R_1$ et/ou $R_2$ représentent un groupement carbamoyle.

Dans le procédé selon l'invention, le groupement A des composés de départ II, peut notamment être soit un reste acétyle, propionyle, butyryle, valéryle ou caproyle, soit un reste d'acide borique, soit un reste furanyle ou pyranyle.

Dans une mise en œuvre avantageuse du procédé de l'invention, le réactif de formule III est préparé, par exemple, par action d'un réactif de formule

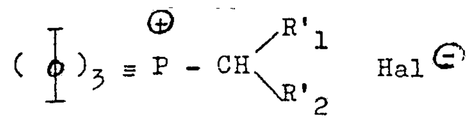

$Hal^{\ominus}$ représentant un anion halogénure, sur une base forte choisie dans le groupe constitué par les hydrures alcalins, les amidures alcalins, les alcoolates alcalins et les alcoylithiens et le solvant utilisé est choisi dans le groupe constitué par l'éther éthylique, le diméthylsulfoxide, le tétrahydrofuran, le diméthoxyéthane, les alcanols, l'éther monoéthylique du diéthylèneglycol et l'éther diéthylique du diéthylèneglycol.

Ces conditions ne sont évidemment pas limitatives du procédé de l'invention.

Le blocage éventuel de l'hydroxyle des composés II peut être effectué par un agent d'acylation tel qu'un anhydride ou un halogénure d'acyle et en présence d'une base tertiaire. Le déblocage final de fonction hydroxyle est effectué par un agent de désacylation tel qu'un agent basique. Le blocage peut également être effectué par l'acide borique.

Ce blocage peut également être effectué sous forme d'éther. On peut ainsi utiliser le blocage par des groupements du type pyranyle ou furanyle. Ce blocage peut être effectué par les méthodes usuelles. Le déblocage final de la fonction éther est effectué en milieu acide.

Dans le cas où l'on veut préparer un composé I dans lequel $R_1$ et/ou $R_2$ sont des groupements carbamoyles, on prépare d'abord le dérivé dans lequel $R_1$ et/ou $R_2$ sont des groupements alcoloxycarbonyles, puis on fait réagir l'ammoniac sur ce composé pour obtenir le dérivé désiré.

Lorsque les groupements $R'_1$ et $R'_2$ des composés III sont des atomes d'halogène, il est commode de préparer in situ le réactif III correspondant, en faisant réagir, au sein d'un solvant en présence d'une base forte, la triphénylphosphine et un haloforme.

On utilise alors, de préférence, comme base forte un alcoolate alcalin et comme solvant un hydrocarbure aliphatique tel que l'heptane.

D'une façon générale ces composés (III) utilisés dans le procédé de l'invention sont préparés, in situ, à partir d'un halogénure de triphénylméthyl phosphonium substitué tel que le bromure ou l'iodure.

L'invention a également pour objet un procédé de préparation des composés de formule I, dans laquelle $R_1$ représente un atome d'hydrogène et $R_2$ représente un groupement cyano, caractérisée en ce que l'on soumet, en présence d'une base forte au sein d'un solvant, un composé de formule II, telle que définie précédemment, à l'action d'un réactif de formule III' :

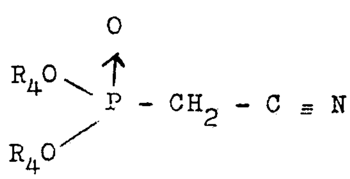

(III')

dans laquelle $R_4$ représente un radical alcoyle renfermant de 1 à 3 atomes de carbone, puis, le cas échéant, débloque la fonction hydroxyle à l'aide d'un agent de désacylation ou de déséthérification, pour obtenir le composé de formule I désiré.

Dans une mise en œuvre avantageuse du procédé ci-dessus, la base forte, le solvant, les agents de blocage et de déblocage de la fonction hydroxyle sont ceux qui ont été énoncés précédemment.

L'invention a également pour objet un procédé de préparation des composés de formule I, dans laquelle $R_1$ et $R_2$, identiques ou différents, représentent un groupement alcoyloxycarbonyle comportant de 2 à 5 atomes de carbone ou un groupement cyano, caractérisée en ce que l'on soumet un composé de formule II, telle que définie précédemment, dans laquelle A représente un atome d'hydrogène, à l'action d'un réactif de formule III"

$$R_1—CH_2—R_2 \qquad (III')$$

dans laquelle $R_1$ et $R_2$ sont définis comme ci-dessus, pour obtenir le composé de formule I désiré.

Dans une mise en œuvre préférentielle du procédé ci-dessus, la réaction est effectuée en présence d'acétate d'ammonium, au sein de l'acide acétique.

Les composés (II) sont, en général, décrits dans la littérature. Lorsque $R_3$ et $R'_3$ représentent de l'hydrogène, il s'agit d'esters ou d'éthers d'alléthrolone ou de l'alléthrolone elle-même. Lorsque $R_3$ ou $R'_3$ sont différents de l'hydrogène, des techniques de préparation analogues à celle décrite dans la partie expérimentale pour le 1 RS-hydroxy 2-méthyl 3-(3-méthyl 2-butényl) 4-oxo cyclopent-2-ène peuvent être utilisées.

L'alléthrolone était déjà connue de longue date comme donnant avec certains acides cyclopropane carboxyliques des esters d'activité insecticide élevée. Ces esters étaient doués d'une forte activité de knock-down mais s'avéraient en général d'une activité létale moins intéressante (voir par exemple le d trans chrysanthémate de dl alléthrolone ou bioalléthrine).

La société demanderesse a maintenant trouvé que les dérivés de l'alléthrolone de formule (I) donnent avec de nombreux acides cyclopropane carboxyliques des esters IV doués vis-à-vis des insectes à la fois d'une activité de knock-down et d'une activité létale élevée, ce qui rend l'utilisation de ces composés particulièrement avantageuse.

Les composés de formule générale (IV), ainsi que leur activité, notamment insecticide, sont décrits dans une demande de brevet déposée en France le même jour que la présente demande par la Société demanderesse et intitulée « Esters de dérivés de l'alléthrolone et d'acides cyclopropane carboxyliques, leur procédé de préparation et les compositions les renfermant » (correspondant à la demande européenne 0018894).

Ces composés (IV) ont pour formule générale :

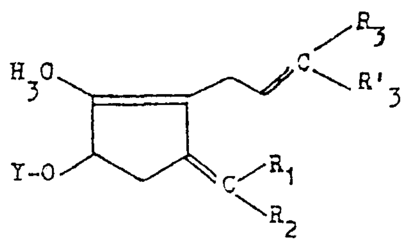

(IV)

dans laquelle $R_1$, $R_2$, $R_3$ et $R'_3$ conservent les significations précitées et Y représente différents restes d'acides et, par exemple, un groupement :

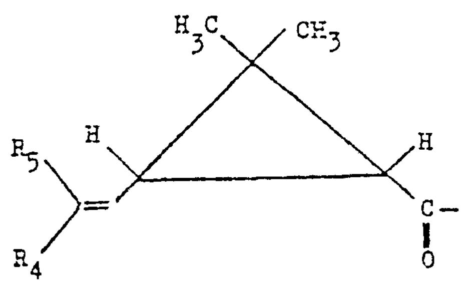

dans lequel $R_5$ et $R_6$, identiques ou différents, représentent notamment un atome de fluor, de chlore ou de brome, ou bien $R_5$ et $R_6$ représentent ensemble avec l'atome de carbone auquel ils sont liés, un homocycle carboné comportant de 3 à 7 atomes de carbone, le cycle cyclopropanique pouvant être de configuration cis ou trans, racémique ou optiquement active, la double liaison en position 1 de la chaîne latérale vinylique pouvant être de configuration (E) ou (Z).

Les composés de formule (IV) sont préparés par estérification d'un composé de formule (I) ou de l'un de ses dérivés fonctionnels, tel qu'un halogénure, à l'aide d'un acide de formule générale (V)

$$Y—OH \qquad (V)$$

dans laquelle Y conserve les significations précipitées, ou bien à l'aide d'un dérivé fonctionnel de cet acide.

Des exemples de ces préparations sont donnés ci-après dans la partie expérimentale.

Les composés (IV) sont actifs, notamment contre les mouches et les moustiques, insectes vis-à-vis desquels ils présentent une activité de knock-down ainsi qu'une activité létale élevée. Les composés de

formule (IV) sont actifs également sur divers insectes, tels que blatella, spodoptera, epilachna, tribolium, sitophilus, aphis.

Des exemples d'activités insecticides de composés de formule (IV) sont donnés plus loin dans la partie expérimentale.

Les exemples suivants illustrent l'invention sans la limiter.

Exemple 1 : 1S-hydroxy 2-méthyl 3-allyl 4-méthylène cyclopent-2-ène.

On mélange sous agitation et sous atmosphère inerte 250 $cm^3$ d'éther, 26,4 $cm^3$ de ter-butanol et 100 g de bromure de triphényl méthyl phosphonium, puis on ajoute en 4 fois et en dix minutes, 31,4 g de ter-butylate de potassium et laisse le mélange réactionnel pendant cinq heures à température ambiante. On refroidit à 0°, + 5 °C et ajoute en vingt minutes 31,9 g de 1S-hydroxy 2-méthyl 3-allyl 4-oxo cyclopent-2-ène dissous dans 30 $cm^3$ d'éther. On maintient le mélange réactionnel pendant deux heures à 0°, + 5 °C, puis le ramène à température ambiante pendant seize heures. On verse sur une solution aqueuse saturée de phosphate monosodique, décante, extrait la phase aqueuse à l'éther, sèche les phases organiques réunies sur sulfate de magnésium et les concentre à sec. On reprend le résidu à l'éther, agite quelques minutes et filtre. On concentre le filtrat sous pression réduite et chromatographie le résidu sur silice en éluant au mélange cyclohexane-acétate d'éthyle (60-40) contenant 2 ‰ de triéthylamine et obtient 26,05 g de produit attendu. Par recristallisation dans l'éther de pétrole (eb : 40-70 °C), on obtient un produit dont les constantes sont les suivantes :

F. 23 °C

$[\alpha]_D^{20} = -110° \pm 2°$ (c = 0,8 %, chloroforme).

Spectre IR (chloroforme)

— Absorption à 3 586 $cm^{-1}$ caractéristique du groupement —OH
— Absorption à 865 $cm^{-1}$ caractéristique du groupement $=CH_2$
— Absorption à 1 637 $cm^{-1}$ caractéristique du groupement $>C=C$
— Absorption à 916 $cm^{-1}$ caractéristique du groupement $-HC=CH_2$

Exemple 2 : 1RS-hydroxy 2-méthyl 3-(3-méthyl 2-butényl) 4-méthylène cyclopent-2-ène.

On met en suspension, sous agitation, 29,7 g de bromure de triphényl méthyl phosphonium, dans 100 ml d'éther et 7,84 ml de ter-butanol, puis ajoute en trente minutes en 6 fois, 9,93 g de ter-butylate de potassium et laisse sous agitation et sous atmosphère inerte pendant cinq heures à 20 °C. On refroidit à 0 °C et ajoute en vingt minutes 10 g de 1RS-hydroxy 2-méthyl 3-(3-méthyl 2-buténγl) 4-oxo cyclopent-2-ène dissous dans 10 ml d'éther. On maintient le milieu réactionnel à 0 °C pendant seize heures sous agitation et sous atmosphère inerte, laisse revenir à 20 °C, agite de nouveau pendant trois heures, verse dans une solution aqueuse saturée de phosphate monosodique et agite pendant quinze minutes. On décante, extrait à l'éther, sèche les phases organiques réunies sur sulfate de magnésium, filtre et amène à sec le filtrat sous pression réduite. On reprend le résidu à l'éther, agite pendant dix minutes à 0 °C, essore le précipité et le lave à l'éther. On amène à sec le filtrat sous pression réduite et obtient 19,54 g de produit attendu brut que l'on purifie par chromatographie sur silice en éluant au mélange benzène-acétate d'éthyle (7-3) contenant 1 ‰ de triéthylamine. On recueille 7,244 g de produit pur de Rf = 0,55.

Spectre IR (chloroforme)

— Absorption complexe à 3 605 $cm^{-1}$-3 580 $cm^{-1}$ caractéristique du groupement —OH
— Absorption à 1 630 $cm^{-1}$ caractéristique de $>C=C<$
— Absorption à 865 $cm^{-1}$ caractéristique de $=CH_2$

Spectre RMN ($CDCl_3$, 60 MHz)

— Pic à 1,7 p.p.m. caractéristique des hydrogènes des groupements $-CH_3$ en bout de chaîne latérale.

— Pic à 1,83 p.p.m. caractéristique des hydrogènes des groupements $-CH_3$ en position 2.

— Pic à 1,98 p.p.m. caractéristique de l'hydrogène du groupement —OH en position 1.

— Pics de 2,83 à 2,95 p.p.m. caractéristiques des hydrogènes en position 1 de la chaîne latérale.

— Pic à 5,03 p.p.m. (triplet J = 7 Hz) caractéristique des hydrogènes en position 3 de la chaîne latérale.

— Pics de 4,42 à 4,83 p.p.m. caractéristiques de l'hydrogène en α du groupement —OH et du méthylène en position 4.

Le 1RS-hydroxy 2-méthyl 3-(2-méthyl 2-buténγl) 4-oxo cyclopent-2-ène utilisé au départ de l'exemple 2 peut être préparé de la manière suivante :

Stade A : 7-méthyl 3-oxo 6-octénoate d'éthyle

On introduit sous agitation et à − 60 °C, 1,4 g de nitrate ferrique dans 1 200 $cm^3$ d'ammoniac liquide, laisse sous agitation pendant cinq minutes, ajoute 2 g de sodium (toujours à − 60 °C) et poursuit l'agitation pendant 10 minutes. On introduit ensuite 104 g de sodium en deux heures trente minutes à − 55° ± 5 °C et agite de nouveau à cette température pendant une heure. Au bout de ce temps, on ajoute au mélange réactionnel en trente minutes et sans dépasser − 30 °C, 300 g d'acétyl acétate d'éthyle, puis 1 000 $cm^3$ d'éther refroidi à − 20 °C et laisse sous agitation pendant cinq minutes. On introduit ensuite en trente minutes sans dépasser − 25 °C, 289 g de 1-chloro 3-méthyl 2-butène, laisse en contact à température ambiante pendant seize heures, ajoute 1 000 $cm^3$ d'éther et progressivement, sans dépasser +15 °C, une solution de 250 $cm^3$ d'acide acétique dans 1 000 $cm^3$ d'eau. On décante, extrait la phase aqueuse à l'éther, lave les phases organiques réunies avec de l'acide chlorhydrique 2N, puis à l'eau, sèche sur sulfate de sodium et amène à sec sous pression réduite. On obtient par rectification du résidu sous pression réduite 175,5 g de produit attendu, Eb (3 mm-Hg) : 98 à 107 °C.

Spectre RMN ($CDCl_3$, 60 MHz)

— Pic à 1,28 p.p.m. (triplet J : 7 Hz). } caractéristiques des hydrogènes du
— Pic à 4,29 p.p.m. (quadruplet J : 7,5 Hz). } groupement $-COOC_2H_5$
— Pic à 3,62 p.p.m. caractéristique des hydrogènes du groupement $-CH_2-$ en position 2.
— Pic à 1,64 p.p.m. (doublet J : 3 Hz) caractéristique des hydrogènes du $-CH_3$ en position 7.
— Pic à 5,08 p.p.m. caractéristique de l'hydrogène en position 6.

Stade B : 3-hydroxy 9-méthyl 8-décène 2,5-dione

On met en suspension 175 g de 7-méthyl 3-oxo 6-octénoate d'éthyle dans 875 $cm^3$ d'eau, puis ajoute en une heure environ sans dépasser + 33 °C, 97 $cm^3$ d'hydroxyde de sodium 10 N et laisse sous agitation pendant vingt heures à température ambiante. On ajoute au mélange réactionnel 25 $cm^3$ d'acide acétique pour amener à pH = 7, puis on introduit en trois heures à + 20°, + 25 °C en maintenant le pH à 7,396 g de pyruvaldéhyde en solution aqueuse (16,8 % en poids) et laisse sous agitation pendant vingt heures à température ambiante (durant ce temps, on a consommé 30 $cm^3$ d'acide acétique pour maintenir le pH à 7). On ajoute ensuite 1 000 $cm^3$ de chlorure de méthylène, agite pendant dix minutes, décante, extrait la phase aqueuse au chlorure de méthylène, sèche les phases organiques sur sulfate de sodium et amène à sec sous pression réduite. On obtient 175 g de produit attendu.

Stade C : 1RS-hydroxy 2-méthyl 3-(3-méthyl 2-buténylㅡ) 4-oxo cyclopent-2-ène

On fait barboter de l'azote pendant une heure dans 875 $cm^3$ d'hydroxyde de sodium 1N, puis introduit 175 mg d'hydroquinone, amène la température à + 3 °C et introduit en une heure à + 2° ± 1 °C, 175 g de 3-hydroxy 9-méthyl 8-décène 2,5-dione. On laisse le mélange réactionnel sous agitation pendant deux heures trente minutes à + 2° ± 1 °C, introduit ensuite, toujours sous agitation, 80 $cm^3$ d'acide chlorhydrique concentré, agite pendant trente minutes en laissant remonter la température à + 20 °C. On ajoute alors 200 g de chlorure de sodium, agite pendant dix minutes, introduit 1 000 $cm^3$ de chlorure de méthylène, décante, extrait la phase aqueuse avec du chlorure de méthylène, sèche les phases organiques réunies sur sulfate de sodium et amène à sec sous pression réduite. On obtient un résidu que l'on rectifie sous pression réduite et recueille 53,4 g de produit cherché.

Eb 0,5 mm Hg : 143 à 148 °C.

Spectre RMN ($CDCl_3$, 60 MHz)

— Pic à 1,72 p.p.m. caractéristique des hydrogènes des groupements $-OH_3$ en bout de chaîne latérale.
— Pic à 2,87 p.p.m. (doublet J : 7 Hz) caractéristique des hydrogènes en position 1 de la chaîne latérale.
— Pic à 4,7 p.p.m. caractéristique de l'hydrogène en $\alpha$ du groupement $-OH$.
— Pic à 5,03 p.p.m. (triplet J : 7 Hz) caractéristique de l'hydrogène en position 2 de la chaîne latérale.
— Pic à 2,67 p.p.m. caractéristique de l'hydrogène du radical $-OH$.
— Pics de 2,08 à 2,92 p.p.m. caractéristiques du groupement $= CH_2$ en position 4 du cyclopentène.

Exemple 3 : 1S-hydroxy 2-méthyl 3-allyl 4-dichlorométhylène cyclopent-2-ène.

Stade A : 1S-acétoxy 2-méthyl 3-allyl 4-oxo cyclopent-2-ène

On dissout 2 g de 1S-hydroxy 2-méthyl 3-allyl 4-oxo cyclopent-2-ène dans 15 ml de chlorure de méthylène, puis ajoute 9,15 ml de triéthylamine et 3,1 ml d'anhydride acétique. Après trente minutes de

réaction, on verse sur une solution aqueuse saturée de phosphate monosodique, extrait à l'éther, lave à l'eau, sèche sur sulfate de magnésium, filtre et amène le filtrat à sec. On obtient 3,049 g de produit attendu.

Spectre RMN ($CDCl_3$, 90 MHz).

— Pics à 1,67 et 2,02 p.p.m. caractéristiques des hydrogènes du radical $—CH_3$ en position 2 et du groupement acétoxy en position 1.

— Pics de 2,11 à 3,01 p.p.m. caractéristiques des hydrogènes en position 5 du cycle et en position 1 de la chaîne latérale.

— Pics de 4,94 à 5,09 p.p.m. caractéristiques des hydrogènes en bout de la chaîne latérale.

— Pics de 5,55 à 6 p.p.m. caractéristiques de l'hydrogène en position 2 de la chaîne latérale.

Stade B : 1S-acétoxy 2-méthyl 3-allyl 4-dichlorométhylène cyclopent-2-ène

On met sous agitation à température ambiante, un mélange de 111 g de triphényl phosphine broyée, 1 000 $cm^3$ d'heptane, 60 $cm^3$ de ter-butanol et 70,8 g de ter-butylate de potassium. On refroidit à − 20 °C et introduit en une heure trente minutes sous agitation, une solution de 75,6 g de chloroforme dans 60 $cm^3$ d'heptane, maintient l'agitation pendant cinq heures à − 20 °C et laisse seize heures au repos à cette température. On obtient une solution d'ylide dont on prélève environ la moitié que l'on conserve sous azote à − 20 °C. A la moitié restante, on ajoute à − 20 °C sous atmosphère inerte et sous agitation, 26 g de 1S-acétoxy 2-méthyl 3-allyl 4-oxo cyclopent-2-ène en solution dans 140 $cm^3$ de tétrahydrofuranne, laisse revenir à température ambiante et agite cinq heures à cette température. On introduit alors à − 20 °C, la 2ème partie d'ylide conservée sous azote à − 20 °C, laisse revenir la température à + 20 °C et agite pendant seize heures à cette température et sous atmosphère inerte. On filtre, ajoute au filtrat une solution aqueuse de phosphate monosodique, extrait à l'éther, sèche les phases organiques réunies sur sulfate de sodium, filtre et amène à sec sous pression réduite. On purifie le résidu par chromatographie sur silice en éluant au mélange benzène acétate d'éthyle (8-2) contenant 1 ‰ de triéthylamine et recueille 14 g de produit attendu Rf = 0,5.

Stade C : 1S-hydroxy 2-méthyl 3-allyl 4-dichlorométhylène cyclopent-2-ène

On dissout 14 g du produit obtenu au stade B dans 350 $cm^3$ d'éthanol, ajoute sous agitation 9,3 g de carbonate de sodium dans 195 $cm^3$ d'eau et un peu de dioxane et agite pendant quatre jours à + 20 °C. On amène à sec, reprend le résidu à l'eau, extrait à l'éther isopropylique, sèche les phases organiques réunies sur sulfate de sodium, filtre et amène à sec sous pression réduite. On recueille 10,9 g de produit attendu.

Spectre IR (chloroforme)

— Absorption à 3 600-3 587 $cm^{-1}$ caractéristique du groupement —OH.

— Absorption à 1 635-1 600 $cm^{-1}$ caractéristique du groupement C = C.

Spectre RMN ($CDCl_3$, 60 MHz)

— Pics de 1,77 à 3,17 p.p.m. caractéristiques des hydrogènes en position 5 du cycle.

— Pic à 1,83 p.p.m. caractéristique du groupement $—CH_3$ en position 2 du cycle.

— Pic à 3,27 p.p.m. (doublet J : 7 Hz) caractéristique des hydrogènes en position 1 de la chaîne latérale.

— Pics de 4,5 à 4,67 p.p.m. caractéristiques de l'hydrogène en position 1 du cycle.

— Pics de 4,83 à 5,25 p.p.m. caractéristiques des hydrogènes en bout de la chaîne latérale.

— Pics de 5,5 à 6,33 p.p.m. caractéristiques des hydrogènes en position 2 de la chaîne latérale.

Exemple 4 : 1S-hydroxy 2-méthyl 3-allyl 4-éthylidène cyclopent-2-ène.

On mélange 30 g de 1S-hydroxy 2-méthyl 3-allyl 4-oxo cyclopent-2-ène avec 200 $cm^3$ de benzène, ajoute 4 g d'acide borique et porte au reflux pendant 5 heures. On distille ensuite 140 $cm^3$ de benzène à pression atmosphérique, puis concentre à sec sous pression réduite. On reprend le résidu dans le benzène jusqu'à obtention d'un volume de 100 $cm^3$.

On mélange, par ailleurs, 68 g de bromure de triphényléthyl phosphonium, 250 $cm^3$ d'éther et 18 $cm^3$ de ter-butanol. On ajoute 20 g de ter-butylate de potassium en 3 fois à 0, + 5 °C. On agite pendant 4 heures, puis ajoute lentement à 0, + 5 °C, 50 $cm^3$ de la solution benzénique ci-dessus. On agite pendant 17 heures à 20 °C, puis verse dans une solution aqueuse saturée de chlorure d'ammonium glacée. On décante, extrait à l'éther, lave les phases éthérées à l'eau salée, sèche et évapore le solvant. On reprend le résidu par 250 $cm^3$ d'éther isopropylique, agite le mélange pendant 2 heures à 20 °C, filtre l'insoluble et

concentre le filtrat à sec. On chromatographie le résidu sur silice en éluant au mélange cyclohexane-acétate d'éthyle 7/3 à 1 ‰ de triéthylamine.

On obtient 6,2 g de produit attendu.

$[\alpha]_D^{20} = -126{,}5° \pm 2{,}5°$ (c = 1 %, éthanol).

Spectre IR (chloroforme)

— Absorption à 1 608 $cm^{-1}$ caractéristique de C = C conjuguée.
— Absorption vers 3 585 $cm^{-1}$ caractéristique de OH (complexe).

Spectre RMN ($CDCl_3$ 60 MHz)

— Pics de 2,08 à 3,17 p.p.m. caractéristiques des hydrogènes en 5.
— Pic à 1,8 p.p.m. caractéristique des hydrogènes du méthyle en 2.
— Pics à 4,55 et 4,63 p.p.m. caractéristiques de l'hydrogène en 1.
— Pics de 2,08 à 3,16 p.p.m. caractéristiques des hydrogènes en 1 de la chaîne allyle.
— Pics de 4,83 à 6,1 p.p.m. caractéristiques des autres hydrogènes de la chaîne allyle et de l'hydrogène en 1 de la chaîne éthylidène.
— Pics à 1,6 et 1,71 p.p.m. caractéristiques des hydrogènes en 2 de la chaîne éthylidène.

Le 1S-hydroxy 2-méthyl 3-allyl 4-éthylidène cyclopent-2-ène peut également être obtenu par une méthode analogue à celle décrite à l'exemple 1, au départ du bromure de triphényléthyl phosphonium au lieu du bromure de triphénylméthylphosphonium.

Exemple 5 : 1S-hydroxy 2-méthyl 3-allyl 4(E)-benzylidène cyclopent-2-ène.

On mélange 22 g de ter-butylate de potassium à 250 $cm^3$ de tétrahydrofuranne, puis ajoute 90 $cm^3$ de ter-butanol. On ajoute ensuite lentement 76,4 g de chlorure de triphényl benzyl phosphonium, agite pendant 1 heure à 23 °C, puis ajoute lentement une solution de 29,8 g de 1S-hydroxy 2-méthyl 3-allyl 4-oxo cyclopent-2-ène dans 10 $cm^3$ de tétrahydruranne. On porte au reflux pendant 24 heures, refroidit à 20 °C, verse dans une solution aqueuse glacée de phosphate monosodique, extrait à l'éther, lave la phase éthérée à l'eau, sèche et évapore le solvant. On obtient 81,5 g de produit brut que l'on chromatographie sur silice en éluant au mélange cyclohexane-acétate d'éthyle (1/1). On obtient 25,5 g de produit attendu. F. $< 50$ °C.

Spectre IR (chloroforme)

— Absorption à 3 600 $cm^{-1}$ caractéristique de OH.
— Absorption à 1 635-1 625 $cm^{-1}$ caractéristique du système conjugué.
— Absorption à 1 597-1 488 $cm^{-1}$ caractéristique des bandes aromatiques.
— Absorption à 999-914 $cm^{-1}$ caractéristique de l'allyle.

Spectre RMN ($CDCl_3$ 60 MHz)

— Pic à 4,66 p.p.m., caractéristique de l'hydrogène en 1.
— Pic à 1,89 p.p.m. caractéristique des hydrogènes du méthyle en 2.
— Pics à 3,03 et 3,12 p.p.m. caractéristiques des hydrogènes 40 en 1 de la chaîne allyle.
— Pics de 4,83 à 5,25 p.p.m. caractéristiques des hydrogènes en 3 de la chaîne allyle.
— Pics de 5,55 à 6,16 p.p.m. caractéristiques des hydrogènes en 2 de la chaîne allyle.
— Pic à 6,28 p.p.m. caractéristique de l'hydrogène du benzylidène.
— Pic à environ 7,32 p.p.m. caractéristique des hydrogènes aromatiques.

Exemple 6 : 1S-hydroxy 2-méthyl 3-allyl 4-fluoro chlorométhylène cyclopent-2-ène.

Stade A : 1S-acétoxy 2-méthyl 3-allyl 4-fluoro chlorométhylène cyclopent-2-ène

On mélange à 20 °C, 100 $cm^3$ de n-heptane, 6,8 g de terbutylate de potassium et 5,7 $cm^3$ de ter-butanol, puis porte à 50 °C pendant 15 minutes. On ajoute ensuite à 20 °C, 15,75 g de triphényl phosphine dans 10 $cm^3$ de n-heptane. On refroidit à 0 °C, puis ajoute lentement une solution de 7,72 g de dichlorofluorométhane dans 75 $cm^3$ de n-heptane. On ajoute ensuite lentement à 0 °C, 9,71 g de 1S-acétoxy 2-méthyl 3-allyl 4-oxo cyclopent-2-ène dans 10 $cm^3$ de tétrahydrofuranne. On maintient sous agitation à 20 °C pendant 4 heures, puis à 50 °C pendant une heure. On concentre le mélange sous pression réduite, reprend le résidu à l'éther, filtre l'insoluble, lave le filtrat avec une solution aqueuse saturée de phosphate monosodique, puis à l'eau, sèche et évapore le solvant. On reprend le résidu à l'éther, filtre et concentre à sec. On chromatographie les 10 g d'huile brute sur silice en éluant au mélange cyclohexane-acétate d'éthyle (95/5) à 1 ‰ de triéthylamine. On obtient 6,75 g de produit attendu.

Spectre IR (chloroforme)

— Absorption à 1 728 $cm^{-1}$ caractéristique de 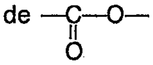

— Absorption à 1 669-1 583 $cm^{-1}$ caractéristique du système conjugué.
— Absorption à 990-919 $cm^{-1}$ caractéristique de $CH = CH_2$.

Stade B : 1S-hydroxy 2-méthyl 3-allyl 4-fluoro chlorométhylène cyclopent-2-ène.

On mélange 6,5 g du produit obtenu au Stade A avec 150 $cm^3$ d'éthanol, puis ajoute 4,5 g de carbonate de sodium dans 90 $cm^3$ d'eau et 20 $cm^3$ de dioxanne. On maintient sous agitation à 20 °C pendant 50 heures, concentre à sec, reprend par 200 $cm^3$ d'eau et extrait à l'éther isopropylique. On sèche la phase éthérée et évapore le solvant. On chromatographie le résidu sur silice en éluant au mélange toluène-acétate d'éthyle (85/15) à 1 ‰ de triéthylamine et obtient 2,5 g de produit attendu.

$[\alpha]_D^{20} = -16{,}5° \pm 2°$ (c = 0,55 %, benzène).
Analyse : $C_{10}H_{12}Cl\ F\ O$ = 202,66
Calculé : C % 59,26 H % 5,96 Cl % 17,49 F % 9,37
Trouvé : C % 59,2 H % 6,0 Cl % 17,7 F % 9,4

Spectre IR (chloroforme)

— Absorption à 3 600 $cm^{-1}$ caractéristique de OH.
— Absorption à 1 668-1 635 $cm^{-1}$ caractéristique de C = C conjuguée.
— Absorption à 990-918 $cm^{-1}$ caractéristique de $CH = CH_2$.

Spectre RMN ($CDCl_3$ 60 MHz)

— Pic à 4,43 p.p.m. caractéristique de hydrogène en 1.
— Pic 1,8 p.p.m. caractéristique des hydrogènes du méthyle en 2.
— Pics de 2,17 à 3,33 p.p.m. caractéristiques des hydrogènes en 1 de la chaîne allyle.
— Pics de 5,5 à 6,5 p.p.m. caractéristiques de l'hydrogène en 2 de la chaîne allyle.
— Pics de 4,8 à 5,3 p.p.m. caractéristiques des hydrogènes en 3 de la chaîne allyle.
— Pics de 2,17 à 3,33 p.p.m. caractéristiques des hydrogènes en 5.

Exemple 7 : 1S-hydroxy 2-méthyl 3-allyl 4-cyanométhylène cyclopent-2-ène.

On introduit 4,8 g d'hydrure de sodium à 50 % dans l'huile minérale, dans 200 $cm^3$ de monoglyme. On ajoute lentement 19,4 $cm^3$ de cyanométhyl phosphonate de O,O-diéthyle. On maintient sous agitation pendant 30 minutes, refroidit à + 5 °C, puis ajoute en 20 minutes 15,22 g de 1S-hydroxy 2-méthyl 3-allyl 4-oxo cyclopent-2-ène dans 20 $cm^3$ de monoéther méthylique de l'éthylène glycol (monoglyme). On maintient ensuite sous agitation à 5 °C pendant 15 minutes, puis à 20 °C pendant 20 heures. On concentre à sec sous pression réduite, reprend le résidu par un mélange à 5 °C, de 100 $cm^3$ d'acide chlorhydrique 1N et 200 $cm^3$ d'eau, extrait à l'éther, lave à l'eau, sèche et évapore le solvant. On obtient 18 g de produit brut que l'on chromatographie sur silice dans le mélange chlorure de méthylène-acétate d'éthyle (95/5), puis dans le mélange toluène-acétate d'éthyle (6/4). On obtient 8,76 g de produit attendu.

$[\alpha]_D^{20} = -140° \pm 3°$ (c = 0,42 %, benzène).
Analyse : $C_{11}H_{13}NO$ : 175,23
Calculé : C % 75,39 H % 7,47 N % 7,99
Trouvé : C % 75,3 H % 7,5 N % 7,8

Spectre IR (chloroforme)

— Absorption à 3 600 $cm^{-1}$ caractéristique de OH.
— Absorption à 2 205 $cm^{-1}$ caractéristique CN conjugué.
— Absorption 1 636 et 1 611 $cm^{-1}$ caractéristique C = C conjuguée.
— Absorption 990 et 919 $cm^{-1}$ caractéristique $CH = CH_2$ (déformation).

Spectre RMN ($CDCl_3$ 60 MHz)

— Pics de 2,33 à 3,5 p.p.m. caractéristiques des hydrogènes en 5.
— Pic à 4,72 p.p.m. caractéristique de l'hydrogène en 1.
— Pic à 1,93 p.p.m. caractéristique des hydrogènes du méthyle en 2.
— Pics de 2,33 à 3,5 p.p.m. caractéristiques des hydrogènes en 1 de la chaîne allyle.
— Pics de 5,42 à 6,08 p.p.m. caractéristiques de l'hydrogène en 2 de la chaîne allyle.

— Pics de 4,75 à 5,33 p.p.m. caractéristiques des hydrogènes en 3 de la chaîne allyle et de l'hydrogène du cyanométhylène.

Exemple 8 : 1S-hydroxy 2-méthyl 3-allyl 4-(cyano éthoxy carbonyl) méthylène cyclopent-2-ène.

On mélange 3,1 g de 1S-hydroxy 2-méthyl 3-allyl 4-oxo méthylène cyclopent-2-ène, 4,52 g de cyanoacétate d'éthyle, 0,7 g d'acétate d'ammonium, 1 $cm^3$ d'acide acétique et 50 $cm^3$ de toluène. On porte au reflux pendant 6 heures, puis élimine l'eau par distillation et refroidit à 20 °C. On ajoute 0,6 g d'acétate d'ammonium dans 1 $cm^3$ d'acide acétique, porte à nouveau au reflux pendant 6 heures, puis élimine à nouveau l'eau formée par distillation. On refroidit à 20 °C, dilue à l'éther, décante, lave la phase éthérée à l'eau, sèche, évapore à sec et obtient 4,15 g de produit brut. On chromatographie ce produit brut sur silice, en éluant au mélange benzène-acétate d'éthyle (6/4) et obtient 1,06 g de produit attendu. $[\alpha]_D^{20} = -87° \pm 2°$ (c = 0,9 %, chloroforme).

Spectre IR (chloroforme)

— Absorption à 3 600 $cm^{-1}$ caractéristique de OH.
— Absorption à 2 220 $cm^{-1}$ caractéristique de C≡N.
— Absorption à 1 718 $cm^{-1}$ caractéristique de C = O ester.
— Absorptions à 1 637, 1 616 et 1 507 $cm^{-1}$ caractéristiques de C = C conjuguée.
— Absorptions à 900-918 $cm^{-1}$ caractéristiques de $CH=CH_2$ (Déformation).

Spectre RMN ($CDCl_3$ 60 MHz)

— Pics à 1,22, 1,33, 1,45, 4,11, 4,23, 4,35 et 4,47 p.p.m. caractéristiques du —COO Et.
— Pic à 2,06 p.p.m. caractéristique des hydrogènes du méthyle en 2.
— Pic à 2,58 p.p.m. caractéristique de l'hydrogène du OH.
— Pics de 2,75 à 4 p.p.m. caractéristiques des hydrogènes en 5.
— Pics de 3,42 à 3,53 p.p.m. caractéristiques des hygrogènes en 1 de la chaîne allyle.
— Pic à 4,75 p.p.m. caractéristique de l'hydrogène en 1.
— Pics de 4,66 à 5,25 p.p.m. caractéristiques des hydrogènes en 3 de la chaîne allyle.
— Pics de 5,67 à 6,25 p.p.m. caractéristiques de l'hydrogène en 2 de la chaîne allyle.

Exemple 9 : 1S-hydroxy 2-méthyl 3-allyl 4-dicyanométhylène cyclopent-2-ène.

On opère de manière analogue à celle décrite à l'exemple 8, au départ de 45 g de 1S-hydroxy 2-méthyl 3-allyl 4-oxo cyclopent-2-ène et de 24 g de malonitrile. On obtient après chromatographie sur silice dans le mélange toluène-acétate d'éthyle (6/4), puis (8/2), 34,9 g de produit attendu.

$[\alpha]_D^{20} = -203° \pm 4°$ (c = 0,5 %, benzène).

Analyse : $C_{12}H_{12}N_2O$

| | | | |
|---|---|---|---|
| Calculé : | C % 71,98 | H % 6,04 | N % 13,99 |
| Trouvé : | C % 71,8 | H % 6,2 | N % 13,8 |

Spectre IR (chloroforme)

— Absorption à 3 600 $cm^{-1}$ caractéristique de OH.
— Absorption à 2 222 $cm^{-1}$ caractéristique de C≡N.
— Absorption à 1 637, 1 611 et 1 567 $cm^{-1}$, caractéristique de C=C conjuguée.
— Absorption à 990 et 920 $cm^{-1}$, caractéristique de $CH=CH_2$ (Déformation).

Spectre RMN ($CDCl_3$ 60 MHz)

— Pic à 4,75 p.p.m. caractéristique de l'hydrogène en 1.
— Pic à 2,58 p.p.m. caractéristique de l'hydrogène du OH.
— Pic à 2,07 p.p.m. caractéristique des hydrogènes du méthyle en 2.
— Pics de 3,25 à 3,42 p.p.m. caractéristiques des hydrogènes en 1 de la chaîne allyle.
— Pics de 5,5 à 6,25 p.p.m. caractéristiques de l'hydrogène en 2 de la chaîne allyle.
— Pics de 4,67 à 5,25 p.p.m. caractéristiques des hydrogènes en 3 de la chaîne allyle.

Exemple 10 : 2,2-diméthyl 3S-(2,2-difluoroéthényl) cyclopropane 1R-carboxylate de (1S) 2-méthyl 3-allyl 4-méthylène cyclopent-2-ène-1-yle.

A une suspension de 3 g de 1S-hydroxy 2-méthyl 3-allyl 4-méthylène cyclopent-2-ène, obtenu à l'exemple 1, dans 30 $cm^3$ de benzène et 4,5 $cm^3$ de pyridine, on ajoute sans dépasser + 30 °C et sous agitation, 3,88 g de chlorure d'acide 2,2-diméthyl 3S-(2,2-difluoroéthényl) cyclopropane-1R-carboxylique

en solution dans 5 $cm^3$ de benzène, agite pendant quatre heures à + 20 °C, puis verse dans l'eau. On décante, extrait à l'éther isopropylique, lave les extraits organiques réunis à l'eau, les sèche sur sulfate de sodium, filtre et amène à sec sous pression réduite. On purifie l'huile résiduelle par rectification sous pression réduite et obtient 2,3 g de produit pur.

Eb 0,1 mm Hg : 113-114 °C.
$[\alpha]_D^{20} = -67° \pm 2,5°$ (c = 0,6 %, benzène)
Analyse : $C_{18}H_{22}F_2O_2$

| | | | |
|---|---|---|---|
| Calculé : | C % 70,11 | H % 7,19 | F % 12,32 |
| Trouvé : | C % 69,8 % | H %7,2 | F %12,5 |

Spectre IR (chloroforme)

— Absorption à 1 746-1 716 $cm^{-1}$ caractéristique des groupements C = O et 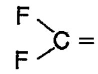

— Absorption à 1 636 $cm^{-1}$ caractéristique des groupements C = C du cyclopentène et = $CH_2$.
— Absorption à 920-991 $cm^{-1}$ caractéristique du groupement CH = $CH_2$.

Exemple 11 : 2,2-diméthyl 3S-(2,2-dichloroéthényl) cyclopropane 1R-carboxylate de (1S) 2-méthyle 3-allyl 4-méthylène cyclopent-2-ène 1-yle.

On ajoute, en quinze minutes, 7 g de chlorure d'acide 2,2 diméthyl 3S-(2,2-dichloroéthényl) cyclopropane-1R-carboxylique dissous dans 5 ml de benzène à une solution de 4,40 g de 1S-hydroxy 2-méthyl 3-allyl 4-méthylène cyclopent-2-ène, obtenu à l'exemple 1, dans 15 $cm^3$ de benzène et 2,6 $cm^3$ de pyridine, puis laisse sous agitation pendant seize heures. On ajoute alors 20 $cm^3$ d'eau, agite cinq minutes, décante, extrait la phase aqueuse au benzène, lave la phase organique à l'eau, extrait les eaux de lavage au benzène, sèche les phases organiques réunies sur sulfate de magnésium, filtre et amène à sec sous pression réduite. On purifie l'huile résiduelle par chromatographie réduite. On purifie l'huile résiduelle par chromatographie sur silice en éluant au mélange cyclohexane-acétate d'éthyle (95-5) contenant 1 ‰ de triéthylamine et recueille 6,99 g de produit attendu pur, Rf : 0,58.

$[\alpha]_D^{20}$ : $-45,5° \pm 1,5°$ (c = 1 %, éthanol)
Analyse : ($C_{18}H_{22}O_2Cl_2$)

| | | | |
|---|---|---|---|
| Calculé : | C % 63,35 | H % 6,49 | Cl % 20,77 |
| Trouvé : | C % 63,3 | H % 6,6 | Cl % 20,3 |

Spectre IR (Chloroforme)

— Absorption à 1 717 $cm^{-1}$ caractéristique du groupement C = O
— Absorptions à 1 633-1 618 $cm^{-1}$ caractéristiques du groupement C = C
— Absorptions à 990-917 $cm^{-1}$ caractéristiques du groupement —CH = $CH_2$

Exemple 12 : 2,2-diméthyl 3R-cyclopentylidène méthyl cyclopropane-1R-carboxylate de (1S) 2-méthyl 3-allyl 4-méthylène cyclopent-2-ène 1-yle.

A une suspension de 5 g de 1S-hydroxy 2-méthyl 3-allyl 4-méthylène cyclopent-2-ène (obtenu à l'exemple 1) dans 15 $cm^3$ de benzène et 2,96 $cm^3$ de pyridine, on ajoute en dix minutes en maintenant la température à + 28 °C, 7,08 g de chlorure de l'acide 2,2-diméthyl 3R-cyclopentylidène méthyl cyclopropane-1R-carboxylique dissous dans 3 $cm^3$ de benzène, laisse sous agitation pendant dix-sept heures à + 20 °C. On ajoute 20 $cm^3$ d'eau, agite dix minutes, décante, extrait la phase aqueuse au benzène, lave les phases organiques à l'eau, extrait les eaux de lavage du benzène, sèche les phases organiques réunies sur sulfate de magnésium, filtre et amène à sec le filtrat sous pression réduite. On purifie le résidu par chromatographie sur silice en éluant au mélange cyclohexane-acétate d'éthyle (95-5) contenant 1 ‰ de triéthylamine et obtient 8,24 g de produit attendu. Rf : 0,55.

$[\alpha]_D^{20} = -87° \pm 1°$ (c = 0,9 %, éthanol).

Spectre IR (chloroforme)

— Absorption à 1 715 $cm^{-1}$ caractéristique du groupement >C = O.
— Absorption à 1 635 $cm^{-1}$ caractéristique du groupement C = C.
— Absorption à 865 $cm^{-1}$ caractéristique du groupement >C = $CH_2$.

Exemple 13 : Etude de l'activité insecticide de quelques composés IV.

A. Protocole des essais insecticides :

1) Activité de knock-down sur mouches domestiques

On opère par pulvérisation directe en chambre de Kearns et March en utilisant comme solvant un mélange en volumes égaux d'acétone et de kérosène (quantité de solution utilisée 2 × 0,2 $cm^3$). On utilise 50 insectes par traitement. On effectue les contrôles, deux, quatre, six, huit, dix et quinze minutes après pulvérisation.

2) Activité létale sur mouches domestiques

On opère par application topique de 1 µl de solution acétonique sur le thorax dorsal des insectes à l'aide du micromanipulateur d'Arnold. On effectue le contrôle de mortalité vingt-quatre heures après traitement.

Les essais peuvent être effectués sans synergiste ou avec addition de butoxyde de pipéronyle (10 parties de synergiste pour une partie de composé à tester).

Les résultats expérimentaux sont exprimés en DL 50 ou dose (en nanogrammes) nécessaire pour tuer 50 % des insectes.

3) Activité létale sur Aedes aegypti

La méthode utilisée dans ce test est la méthode O.M.S. 20 insectes adultes sont mis en contact avec le papier traité. Le traitement est effectué en déposant, à la pipette, de la solution acétonique du produit à tester sur une feuille de papier filtre de 180 $cm^2$. La contamination est faite vingt-quatre heures après le traitement du papier.

4) Activité de knoch-down sur Aedes aegypti

La méthode utilisée est analogue à celle décrite précédemment pour l'effet létal mais le comptage des insectes abattus est effectué à intervalles rapprochés (2 minutes, 4 minutes...) jusqu'à ce qu'on ait observé que tous les insectes sont abattus.

5) Activité insecticide sur Aphis Craccivora.

On opère par application topique d'une microgoutte de solution acétonique du composé à tester. Les DL 50 sont exprimées en nanogramme par insecte.

6) Activité insecticide sur Spodoptera littoralis

Les essais sont effectués par application topique. On dépose 1 µl d'une solution acétonique du produit à tester sur le thorax dorsal de chaque individu (sauf mention particulière). On utilise 15 chenilles de Spodoptera littoralis au 4e stade larvaire pour chaque dose employée. Après traitement, les individus sont placés sur un milieu nutritif artificiel (milieu de Poitout). On effectue le contrôle d'efficacité vingt-quatre heures, quarante-huit heures après traitement et l'on détermine la $DL_{50}$ en nanogramme par insecte.

7) Activité insecticide sur Sitophilus granarius

On opère par application topique de manière analogue à celle utilisée par les larves de Spodoptera. Après traitement les insectes sont conservés sur grain de blé. Les observations de mortalité sont effectuées vingt-quatre heures, quarante-huit heures six et sept jours après traitement.

B. Résultats :

I. Activité insecticide du 2,2 diméthyl 3R-cyclopentylidène méthyl cyclopropane-1R-carboxylate de (1S) 2-méthyl 3-allyl 4-méthylène cyclopent-2-ène 1-yle (composé A).

1) Activité de knock-down sur mouches

Sans synergiste le $KT_{50}$ du composé A a été trouvé égal à onze minutes et avec synergiste à 9,5 minutes.

2) Activité létale sur mouches

La $DL_{50}$ du composé A a été trouvée égale à 5,19 nanogrammes, par insecte.

3) Activité létale sur Aedes aegypti

La $DL_{50}$ du composé A a été atteinte pour un dépôt de 0,75 $mg/m^2$.

4) Activité de knock-down Aedes aegypti

Au bout de quatre minutes il y a 31,9 % d'insectes abattus, au bout de six minutes 76,5 % et au bout de huit minutes 100 % (pour une dose de 83 mg par $m^2$).

5) Activité sur Aphis Craccivora

A la dose de 2 nanogrammes par insecte, au bout de vingt-quatre heures et quarante-huit heures, la mortalité est de 100 %.

6) Activité sur Spodoptera littoralis

A la dose de 10 nanogrammes par insecte au bout de vingt-quatre heures, la mortalité est de 70 % et au bout de quarante-huit heures de 100 %.

7) Activité sur Sitophilus granarius

A la dose de 20 nanogrammes par insecte, la mortalité est voisine de 100 % au bout de vingt-quatre heures.

8) Conclusion

Le composé A est doué d'une intéressante activité insecticide.

II. Activité insecticide du 2,2-diméthyl 3S-(2,2-difluoroéthényl) cyclopropane-1R-carboxylate de (1S) 2-méthyl 3-allyl 4-méthylène cyclopent-2-ène 1-yle (composé B).

1) Activité de knock-down sur mouche

Le $KT_{50}$ a été trouvé égal à 3,6 minutes par insecte sans addition de synergiste. Dans les mêmes conditions le $KT_{50}$ de la bioalléthrine a été trouvé à 9,8.

2) Activité létale sur mouche

La $DL_{50}$ a été trouvée égale à 2,7 nanogrammes par insecte sans synergiste et à 2,8 avec addition de butoxyde de pipéronyle.

3) Activité insecticide sur Epilachna Varivestris

Le protocole opératoire est analogue à celui utilisé pour Spodoptera littoralis. La $DL_{50}$ a été trouvée égale à 4,5-nanogrammes par insecte.

4) Conclusion

Le composé B est doué d'une intéressante activité insecticide.

**Revendications**

1. Les composés de formule générale I :

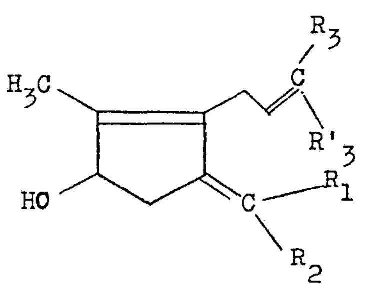

(I)

dans laquelle $R_1$ et $R_2$, identiques ou différents, représentent un groupement carbamoyle ou bien des groupements $R'_1$ et $R'_2$, lesquels, identiques ou différents, représentent un atome d'hydrogène, un atome d'halogène, un radical alcoyle comportant de 1 à 6 atomes de carbone, un radical aryle comportant de 6 à 10 atomes de carbone, un radical aralcoyle comportant de 7 à 13 atomes de carbone, un groupement alcoyloxycarbonyle comportant de 2 à 5 atomes de carbone ou un groupement cyano, $R_3$ et $R'_3$, identiques ou différents, représentent un atome d'hydrogène ou un radical alcoyle saturé ou non, comportant de 1 à 3 atomes de carbone, ces composés de formule (I) pouvant être de configuration S, R, ou racémique.

2. Les composés de formule I dont les noms suivent :

— le 1S-hydroxy 2-méthyl 3-allyl 4-méthylène cyclopent-2-ène,
— le 1RS-hydroxy 2-méthyl 3-(3-méthyl 2-buténył) 4-méthylène cyclopent-2-ène,
— le 1S-hydroxy 2-méthyl 3-allyl 4-dichlorométhylène cyclopent-2-ène,
— le 1S-hydroxy 2-méthyl 3-allyl 4-dicyanométhylène cyclopent-2-ène,
— le 1S-hydroxy 2-méthyl 3-allyl 4-éthylidène cyclopent-2-ène,
— le 1S-hydroxy 2-méthyl 3-allyl 4E-benzylidène cyclopent-2-ène,
— le 1S-hydroxy 2-méthyl 3-allyl 4-fluorochlorométhylène cyclopent-2-ène,
— le 1S-hydroxy 2-méthyl 3-allyl 4-cyanométhylène cyclopent-2-ène.
— le 1S-hydroxy 2-méthyl 3-allyl 4-(cyanoéthoxycarbonyl) méthylène cyclopent-2-ène.

3. Procédé de prépration des composés de formule générale I, tels que définis à la revendication 1, caractérisé en ce que l'on soumet, au sein d'un solvant, un composé de formule générale II :

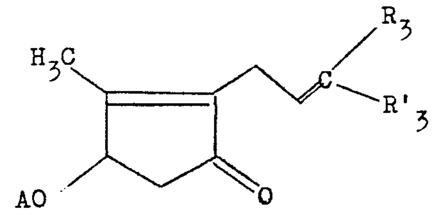

(II)

dans laquelle $R_3$ et $R'_3$ conservent les significations précitées et A représente un atome d'hydrogène, un reste d'acide carboxylique aliphatique comportant de 2 à 6 atomes de carbone, un reste d'acide borique ou un reste d'éther comportant de 2 à 6 atomes de carbone, à l'action d'un réactif de formule III :

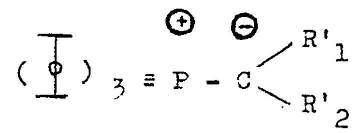

(III)

dans laquelle $R'_1$ et $R'_2$ conservent les significations précitées, débloque, le cas échéant, la fonction hydroxyle, à l'aide d'un agent de désacylation ou de déséthérification, pour obtenir un composé de formule I désiré, dans lequel $R_1$ et $R_2$ ont les valeurs de $R'_1$ et $R'_2$, $R'_1$ et $R'_2$ étant définis comme ci-dessus, puis, le cas échéant, fait réagir un composé I dans lequel $R'_1$ et/ou $R'_2$ représentent un groupement alcoyloxycarbonyle comportant de 2 à 5 atomes de carbone et dans lequel le groupement hydroxyle est éventuellement bloqué par un reste d'acide borique ou d'acide carboxylique aliphatique comportant de 2 à 6 atomes de carbone ou par un reste d'éther comportant de 2 à 6 atomes de carbone, avec l'ammoniac, puis débloque, le cas échéant, le groupement hydroxyle pour obtenir le composé I désiré, dans lequel $R_1$ et/ou $R_2$ représentent un groupement carbamoyle.

4. Procédé selon la revendication 3, caractérisé en ce que le réactif de formule III est préparé, par action d'un réactif de formule

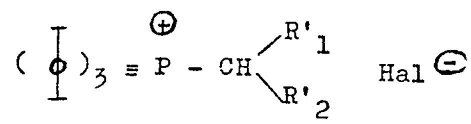

$Hal^{\ominus}$ représentant un anion halogénure, sur une base forte choisie dans le groupe constitué par les hydrures alcalins, les amidures alcalins, les alcoolates alcalins et les alcoyllithiens et en ce que le solvant utilisé est choisi dans le groupe constitué par l'éther éthylique, le diméthylsulfoxyde, le tétrahydrofuran, le diméthoxyéthane, les alcanols, l'éther monométhylique du diéthylèneglycol et l'éther diéthylique du diéthylèneglycol.

5. Procédé selon la revendication 3, caractérisé en ce que le blocage de l'hydroxyle des composés de formule II est effectué par un agent d'acylation tel qu'un anhydride d'acide et en présence d'une base tertiaire.

6. Procédé selon la revendication 3, caractérisé en ce que le déblocage final de la fonction hydroxyle est effectué par un agent de désacylation, tel qu'un agent basique.

7. Procédé selon la revendication 3, caractérisé en ce que dans le cas où $R'_1$ et/ou $R'_2$ représentent un atome d'halogène, le réactif de formule III est obtenu in situ en faisant réagir, au sein d'un solvant et en présence d'une base forte, la triphényl phosphine et un haloforme.

8. Procédé selon la revendication 7, caractérisé en ce que l'on fait réagir la triphényl phosphine et l'haloforme en présence d'un alcoolate alcalin et au sein d'un hydrocarbure aliphatique, tel que l'heptane.

9. Procédé pour la préparation des composés de formule I, dans laquelle $R_1$ représente un atome d'hydrogène et $R_2$ représente un groupement cyano, caractérisée en ce que l'on soumet, en présence d'une base forte, au sein d'un solvant, un composé de formule II, telle que définie à la revendication 3, à l'action d'un réactif de formule III' :

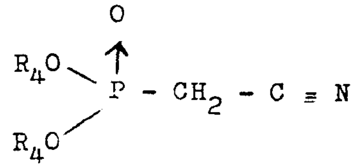

(III')

dans laquelle $R_4$ représente un radical alcoyle renfermant de 1 à 3 atomes de carbone, puis, le cas échéant, débloque la fonction hydroxyle à l'aide d'un agent de désacylation ou de deséthérification, pour obtenir le composé de formule I désiré.

10. Procédé selon la revendication 9, caractérisé en ce que la base forte et le solvant utilisés sont tels que définis à la revendication 4.

11. Procédé pour la préparation des composés de formule I, dans laquelle $R_1$ et $R_2$, identiques ou différents, représentent un groupement alcoyloxycarbonyle comportant de 2 à 5 atomes de carbone ou un groupement cyano, caractérisée en ce que l'on soumet un composé de formule II, telle que définie à la revendication 3, dans laquelle A représente un atome d'hydrogène, à l'action d'un réactif de formule III"

$$R_1—CH_2—R_2 \qquad (III'')$$

dans laquelle $R_1$ et $R_2$ sont définis comme ci-dessus, pour obtenir le composé de formule I désiré.

12. Procédé selon la revendication 11, caractérisé en ce que la réaction est effectuée en présence d'acétate d'ammonium, au sein de l'acide acétique.

**Claims**

1. Compounds with the general formula I

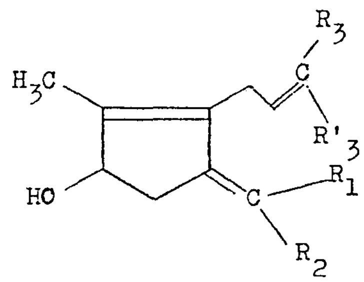

(I)

in which $R_1$ and $R_2$, identical or different, represent carbamyl groups or groups $R'_1$ and $R'_2$, identical or different, each of which represents a hydrogen atom, a halogen atom, an alkyl radical containing 1 to 6 carbon atoms, an aryl radical containing 6 to 10 carbon atoms, an aralkyl radical containing 7 to 13 carbon atoms, an alkyloxycarbonyl group containing 2 to 5 carbon atoms or a cyano group, $R_3$ and $R'_3$, identical or different each represents a hydrogen atom or a saturated or unsaturated alkyl radical containing 1 to 3 carbon atoms, these compounds with the formula (1) being able to be of configuration S, R or racemic.

2. Compounds with the formula I with the following names :

— 1S-hydroxy 2-methyl 3-allyl 4-methylene cyclopent-2-ene,
— 1RS-hydroxy 2-methyl 3-(3-methyl 2-butenyl) 4-methylene cyclopent-2-ene,
— 1S-hydroxy 2-methyl 3-allyl 4-dichloromethylene cyclopent-2-ene,
— 1S-hydroxy 2-methyl 3-allyl 4-dicyanomethylene cyclopent-2-ene,
— 1S-hydroxy 2-methyl 3-allyl 4-ethylidene cyclopent-2-ene,
— 1S-hydroxy 2-methyl 3-allyl 4E-benzylidene cyclopent-2-ene,
— 1S-hydroxy 2-methyl 3-allyl 4-fluorochloromethylene cyclopent-2-ene,
— 1S-hydroxy 2-methyl 3-allyl 4-cyanomethylene cyclopent-2-ene,
— 1S-hydroxy 2-methyl 3-allyl 4-(cyanoethoxycarbonyl) methylene cyclopent-2-ene,

3. Preparation process for compounds with the formula I as defined in Claim 1, characterised in that a compound with the general formula II

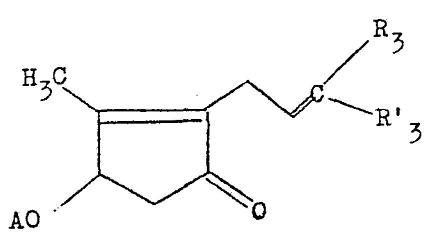

(II)

in which $R_3$ and $R'_3$ retain the previously stated significances and A represents a hydrogen atom, an aliphatic carboxylic acid residue containing 2 to 6 carbon atoms, a boric acid residue or a residue of an ether containing from 2 to 6 carbon atoms, is submitted to the action in a solvent of a reagent with the formula III

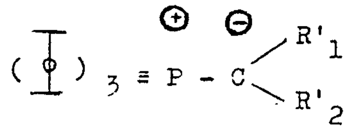

(III)

in which $R'_1$ and $R'_2$ retain the previously stated significances, unblocking, if necessary, the hydroxyl function by means of a de-acylation or de-etherification agent, so as to obtain a compound with the desired formula I in which $R_1$ and $R_2$ have the values of $R'_1$ and $R'_2$, $R'_1$ and $R'_2$ being defined as above, and then, if required, make a compound I, in which $R'_1$ and/or $R'_2$ each represents an alkyloxycarbonyl group containing from 2 to 5 carbon atoms and in which the hydroxyl group is possibly blocked by a residue of boric acid or of an aliphatic carboxylic acid containing from 2 to 6 carbon atoms or by an ether residue containing 2 to 6 carbon atoms, react with ammonia, then, if necessary, unblocking the hydroxyl group so as to obtain the desired group I in which $R_1$ and/or $R_2$ each represent a carbamyl group.

4. Process according to Claim 3, characterised in that the reagent of formula III is prepared by the action of a reagent with the formula

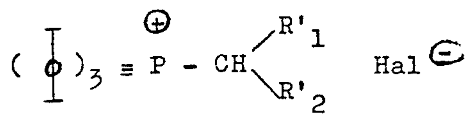

$Hal^{\ominus}$ representing a halide anion, on a strong base chosen from the group constituted by the alkaline hydrides, the alkaline amides, the alkaline alcoholates and the alkyllithiums, and in that the solvent utilized is chosen from the group constituted by ethyl ether, dimethylsulphoxide, tetrahydrofuran, dimethoxyethane, the alkanols, the monomethyl ether of diethyleneglycol and the diethyl ether of diethyleneglycol.

5. Process according to Claim 3, characterised in that the blocking of the hydroxyl of the compounds with the formula II is done by an acylation agent such as an acid anhydride, in the presence of a tertiary base.

6. Process according to Claim 3 characterised in that the final unblocking of the hydroxyl function is done by a de-acylation agent, such as a basic agent.

7. Process according to Claim 3, characterised in that in the case where $R'_1$ and/or $R'_2$ each represent a halogen atom, the reagent with the formula III is obtained in situ by making triphenyl phosphine and a haloform react in a solvent in the presence of a strong base.

8. Process according to Claim 7, characterised in that the triphenyl phosphine and the haloform are made to react in the presence of an alkaline alcoholate and in an aliphatic hydrocarbon, such as heptane.

9. Process for the preparation of the compounds with the formula I in which $R_1$ represents a hydrogen atom and $R_2$ represents a cyano group, characterised in that a compound with the formula II as defined in Claim 3 is submitted to the action of a reagent with the formula III'

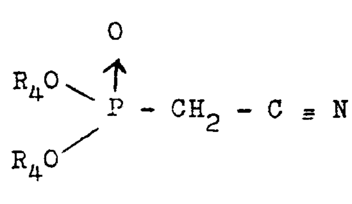

(III')

in a solvent in the presence of a strong base, in which formula $R_4$ represents an alkyl radical containing from 1 to 3 carbon atoms, and then, if necessary the hydroxyl function is unblocked by an agent of de-acylation or de-etherification, so as to obtain the desired compound with the formula I.

10. Process according to Claim 9, characterised in that the strong base and the solvent are as defined in Claim 4.

11. Process for the preparation of the compounds with the formula I, in which $R_1$ and $R_2$, identical or different, each represent an alkyloxycarbonyl group containing from 2 to 5 carbon atoms or a cyano group, characterised in that a compound with the formula II, as defined in Claim 3, in which A represents a hydrogen atom, is submitted to the action of a reagent with the formula III"

$$R_1—CH_2—R_2 \qquad (III")$$

in which $R_1$ and $R_2$ are defined as above, so as to obtain the desired compound with the formula I.

12. Process according to Claim 11, characterised in that the reaction is carried out in acetic acid in the presence of ammonium acetate.

**Ansprüche**

1. Die Verbindungen der allgemeinen Formel I

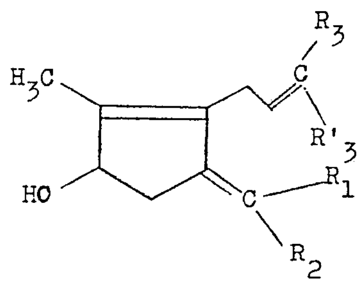

(I)

worin $R_1$ und $R_2$, die gleich oder verschieden sein können, eine Carbamoylgruppe oder Gruppen $R'_1$ und $R'_2$ bedeuten, die gleich oder verschieden sein können und ein Wasserstoffatom, ein Halogenatom, einen Alkylrest mit 1 bis 6 Kohlenstoffatomen, einen Arylrest mit 6 bis 10 Kohlenstoffatomen, einen Aralkylrest mit 7 bis 13 Kohlenstoffatomen, eine Alkoxycarbonylgruppe mit 2 bis 5 Kohlenstoffatomen oder eine Cyanogruppe bedeuten, $R_3$ und $R'_3$, die gleich oder verschieden sein können, ein Wasserstoffatom oder einen gesättigten oder ungesättigten Alkylrest mit 1 bis 3 Kohlenstoffatomen bedeuten, wobei diese Verbindungen der Formel I in S-, R- oder racemischer Konfiguration vorliegen können.

2. Die Verbindungen der Formel I mit den folgenden Bezeichnungen :
— 1S-Hydroxy-2-methyl-3-allyl-4-methylencyclopent-2-en,
— 1RS-Hydroxy-2-methyl-3-(3-methyl-2-butenyl)-4-methylencyclopent-2-en,
— 1S-Hydroxy-2-methyl-3-allyl-4-dichlormethylencyclopent-2-en,
— 1S-Hydroxy-2-methyl-3-allyl-4-dicyanomethylencyclopent-2-en,
— 1S-Hydroxy-2-methyl-3-allyl-4-äthylidencyclopent-2-en,
— 1S-Hydroxy-2-methyl-3-allyl-4E-benzylidencyclopent-2-en,
— 1S-Hydroxy-2-methyl-3-allyl-4-fluorchlormethylencyclopent-2-en,
— 1S-Hydroxy-2-methyl-3-allyl-4-cyanomethylencyclopent-2-en
— 1S-Hydroxy-2-methyl-3-allyl-4-(cyanoäthoxycarbonyl)-methylencyclopent-2-en.

3. Verfahren zur Herstellung der Verbindungen der allgemeinen Formel I gemäß Anspruch 1, dadurch gekennzeichnet, daß man in dem Medium eines Lösungsmittels eine Verbindung der allgemeinen Formel II

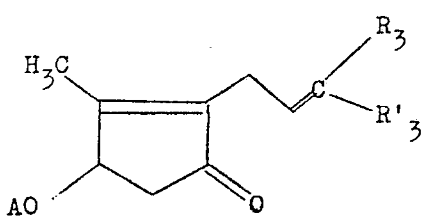

(II)

worin $R_3$ und $R'_3$ die vorstehend angegebenen Bedeutungen besitzen und A ein Wasserstoffatom, einen Rest einer aliphatischen Carbonsäure mit 2 bis 6 Kohlenstoffatomen, einen Rest der Borsäure oder einen Rest eines Äthers mit 2 bis 6 Kohlenstoffatomen bedeutet, der Einwirkung eines Reagens der Formel III

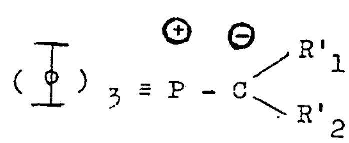

(III)

worin $R'_1$ und $R'_2$ die vorstehend angegebene Bedeutung besitzen, unterzieht, gegebenenfalls die Hydroxylfunktion mit Hilfe eines Entacylierungsmittels oder eines Äther-spaltenden Mittels freisetzt, um eine gewünschte Verbindung der Formel I zu erhalten, worin $R_1$ und $R_2$ die Bedeutungen von $R'_1$ und

$R'_2$ besitzen, wobei $R'_1$ und $R'_2$ wie vorstehend definiert sind, danach gegebenenfalls eine Verbindung I, worin $R'_1$ und/oder $R'_2$ eine Alkoxycarbonylgruppe mit 2 bis 5 Kohlenstoffatomen bedeuten und worin die Hydroxylgruppe gegebenenfalls durch einen Rest der Borsäure oder einer aliphatischen Carbonsäure mit 2 bis 6 Kohlenstoffatomen oder durch einen Rest eines Äthers mit 2 bis 6 Kohlenstoffatomen blockiert ist, mit Ammoniak umsetzt und danach gegebenfalls die Hydroxylgruppe freisetzt, um die gewünschte Verbindung I, worin $R_1$ und/oder $R_2$ eine Carbamoylgruppe bedeuten, zu erhalten.

4. Verfahren gemäß Anspruch 3, dadurch gekennzeichnet, daß das Reagens der Formel III durch Einwirken eines Reagens der Formel

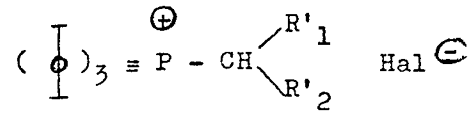

worin Hal$^{\ominus}$ ein Halogenidanion bedeutet, auf ein starke Base, ausgewählt unter den Alkalihydriden, den Alkaliamiden, den Alkalialkoholaten und den Alkyllithiumverbindungen hergestellt wird, und daß das verwendete Lösungsmittel ausgewählt wird unter Äthyläther, Dimethylsulfoxid, Tetrahydrofuran, Dimethoxyäthan, den Alkanolen, dem Monomethyläther von Diäthylenglykol, dem Diäthyläther von Diäthylenglykol.

5. Verfahren gemäß Anspruch 3, dadurch gekennzeichnet, daß die Blockierung der Hydroxylgruppe der Verbindungen der Formel II durch ein Acylierungsmittel wie ein Säureanhydrid und in Gegenwart einer tertiären Base durchgeführt wird.

6. Verfahren gemäß Anspruch 3, dadurch gekennzeichnet, daß die letzendliche Deblockierung der Hydroxylfunktion durch ein Entacylierungsmittel wie ein basisches Mittel bewirkt wird.

7. Verfahren gemäß Anspruch 3, dadurch gekennzeichnet, daß wenn $R'_1$ und/oder $R'_2$ ein Halogenatom bedeuten, das Reagens der Formel III in situ erhalten wird, indem man in dem Medium eines Lösungsmittels und in Gegenwart einer starken Base Triphenylphosphin und ein Haloform umsetzt.

8. Verfahren gemäß Anspruch 7, dadurch gekennzeichnet, daß man Triphenylphosphin und das Haloform in Gegenwart eines Alkalialkoholats oder in dem Medium eines aliphatischen Kohlenwasserstoffs wie Heptan umsetzt.

9. Verfahren zur Herstellung der Verbindungen der Formel I, worin $R_1$ ein Wasserstoffatom bedeutet und $R_2$ eine Cyanogruppe bedeutet, dadurch gekennzeichnet, daß man in Gegenwart einer starken Base in dem Medium eines Lösungsmittels eine Verbindung der Formel II, die wie Anspruch 3 definiert ist, der Einwirkung eines Reagens der Formel III'

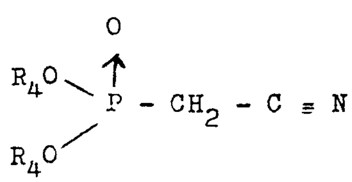

(III')

worin $R_4$ einen Alkylrest mit 1 bis 3 Kohlenstoffatomen bedeutet unterzieht und danach gegebenenfalls die Hydroxylfunktion mit Hilfe eines Entacylierungsmittels oder eines Äther-spaltenden Mittels freisetzt, um die gewünschte Verbindung der Formel I zu erhalten.

10. Verfahren gemäß Anspruch 9, dadurch gekennzeichnet, daß die starke Base und das verwendete Lösungsmittel wie in Anspruch 4 definiert sind.

11. Verfahren zur Herstellung der Verbindungen der Formel I, worin $R_1$ oder $R_2$, die gleich oder verschieden sein können, eine Alkoxycarbonylgruppe mit 2 bis 5 Kohlenstoffatomen oder eine Cyanogruppe bedeuten, dadurch gekennzeichnet, daß man eine Verbindung der Formel II, wie in Anspruch 3 definiert, worin A ein Wasserstoffatom bedeutet, der Einwirkung eines Reagens der Formel III''

$$R_1—CH_2—R_2 \quad \text{(III'')}$$

unterzieht, worin $R_1$ und $R_2$ wie vorstehend definiert sind, um die gewünschte Verbindung der Formel I zu erhalten.

12. Verfahren gemäß Anspruch 11, dadurch gekennzeichnet, daß die Umsetzung in Gegenwart von Ammoniumacetat in dem Medium von Essigsäure durchgeführt wird.